# FASCICULE DE BREVET EUROPEEN

(11) **EP 4 108 191 B1**
(45) Date de publication et mention de la délivrance du brevet: **10.04.2024**
(21) Numéro de dépôt: 22181355.3
(22) Date de dépôt: 27.06.2022
(51) Int. Cl.: A61B 17/50, B26B 11/00

(54) **COUTEAU DE CHASSE OU DE SURVIE DOTÉ D'UN DISPOSITIF D EXTRACTION DE TIQUE**
JAGD- ODER SURVIVALMESSER MIT EINER VORRICHTUNG ZUM HERAUSZIEHEN VON ZECKEN
HUNTING OR SURVIVAL KNIFE PROVIDED WITH A DEVICE FOR EXTRACTING TICKS

(30) Priorité: 25.06.2021 FR 2106870
(43) Date de publication de la demande: 28.12.2022
(73) Titulaire: Opinel SAS, 73000 Chambery (FR)
(72) Inventeur: OPINEL, François, 73000 CHAMBERY (FR)
(74) Mandataire: Germain Maureau

(56) Documents cités:
- WO-A1-2019/077403
- DE-A1-102019 210 459
- DE-U1-202010 014 741
- FR-A1- 2 928 534
- GB-A- 2 437 907

## Description

La présente invention concerne le domaine des couteaux de chasse ou de survie. En particulier, l'invention porte sur un couteau de chasse ou de survie, doté d'un dispositif d'extraction de tique pour extraire de façon sécuritaire une tique de la peau des animaux ou de l'homme.

Les tiques se fixent dans la peau grâce à un rostre (ou tête) qui pénètre l'épiderme. Le retrait de ces tiques se fait traditionnellement avec des pinces de conception variée, mais il arrive souvent que le rostre de la tique reste planté dans la peau. En effet, selon la conception des pinces, le mode de préhension de la tique varie et certaines pinces difficiles à manipuler contribuent à comprimer le corps de la tique. Cette compression favorise la régurgitation de sang et de salive vers la peau de l'hôte. Une infection est alors possible, notamment par transmission de la bactérie « borrelia » à l'origine de la maladie de Lyme. Par ailleurs, selon l'espèce de tiques concernée, la taille de la tique varie et peu de pinces existantes propose une adaptation à la taille de la tique. Or, une pince dont l'ouverture est trop petite risque de conduire à la compression du corps de la tique, ce qui augmente le risque de transmission de maladies. De plus, il existe peu de boitiers de rangement pour pince qui permettraient de transporter la pince facilement, lors d'une sortie en extérieur, tout en limitant les risques de la perdre. Les documents DE202010014741 U1 et WO2019/077403 A1 divulguent des couteaux de poche multifonctionnels dont un des outils est un tire-tique. Le document DE102019210459 A1 décrit un dispositif d'extraction de tique comprenant à une extrémité un dispositif de préhension, pouvant notamment être constitué d'une pince et à l'autre extrémité opposée, un dispositif de réception pouvant comprendre plusieurs fentes configurées pour l'insertion d'une tique.

Aussi, l'un des buts de la présente invention vise à pallier au moins l'un des inconvénients précités. A cet effet, l'invention propose un couteau de chasse ou de survie, tel que défini dans la revendication 1, comprenant un manche et un dispositif d'extraction de tique destiné à extraire une tique de la peau des animaux ou de l'homme, le dispositif d'extraction de tique étant logé de façon amovible dans le manche, en étant mobile entre une position de rangement dans laquelle le dispositif d'extraction de tique est logé à l'intérieur du manche et une position d'utilisation dans laquelle le dispositif d'extraction de tique s'étend hors du manche, le dispositif d'extraction de tique comprenant une portion d'extrémité sur laquelle sont juxtaposés :
un premier organe de retrait de tique comprenant une première fente configurée pour enserrer une tique d'une première taille en vue de son retrait,
un deuxième organe de retrait de tique comprenant une deuxième fente configurée pour enserrer une tique d'une deuxième taille en vue de son retrait, de telle sorte que les premier et deuxième organes de retrait de tique sont aptes au retrait de tiques de tailles différentes.

Ainsi configuré, le couteau de l'invention est multifonctionnel. Il dispose d'une lame et d'un logement pour un dispositif d'extraction de tique, limitant les risques de perte du dispositif d'extraction de tique lors de sortie en extérieur. Le dispositif d'extraction de tique est facile à ranger et à retrouver dans une besace. Aussi, l'utilisateur n'a plus la crainte d'emporter le dispositif d'extraction de tique avec soi lors de sorties et les éventuelles tiques peuvent retirées plus rapidement. Le dispositif d'extraction de tique comprenant une première et une deuxième fentes configurées pour le retrait de tique de grosseurs différentes, le dispositif limite les risques de compression de cette dernière. Il est possible de saisir la tique selon sa grosseur, par la fente appropriée, puis de l'extraire complètement par un mouvement de rotation du dispositif d'extraction.

Selon une disposition, les deux fentes présentent deux tailles différentes.

De manière concrète, la première fente et la deuxième fente comprennent respectivement une première zone d'insertion configurée pour l'insertion d'une tique d'une première taille et une deuxième zone d'insertion configurée pour l'insertion d'une tique d'une deuxième taille, différente de la première taille, la deuxième zone d'insertion étant opposée à la première zone d'insertion.

Selon une possibilité, la première fente et la deuxième fente présentent respectivement une première forme en V et une deuxième forme en V, s'étendant dans deux directions opposées.

Les première et deuxième zones d'insertion sont respectivement formées par une première extrémité libre et une deuxième extrémité libre respectivement de la première forme en V et de la deuxième forme en V. La largeur de la première fente et la largeur de la deuxième fente s'agrandissent vers l'extrémité libre respectivement de la première fente et de la deuxième fente. Ainsi, chacune des fentes présentent une largeur variable permettant l'insertion d'une tique depuis la première ou la deuxième zone d'insertion, vers la pointe de la première forme en V (opposée à la première extrémité libre) ou celle de la deuxième forme en V, sans risquer de comprimer la tique.

Selon une disposition, la première forme en V et de la deuxième forme en V présentent respectivement une première bissectrice et une deuxième bissectrice, la première bissectrice et la deuxième bissectrice étant alignées.

Selon une possibilité, la première bissectrice et la deuxième bissectrice s'étendent dans deux directions opposées.

Selon une disposition, le dispositif d'extraction de tique comprend une portion de préhension, un premier bras de liaison et un deuxième bras de liaison reliant le premier organe de retrait de tique et le deuxième organe de retrait de tique à la portion de préhension.

Selon une disposition, le premier organe de retrait de tique comprend une première dent primaire et une deuxième dent primaire délimitant la première fente et le deuxième organe de retrait de tique comprend une première dent secondaire et une deuxième dent secondaire délimitant la deuxième fente. Les première et deuxième dents primaires ou les première et deuxième dents secondaires permettent de retenir la tique le temps de l'éliminer.

Selon une réalisation particulière, la première dent primaire, la deuxième dent primaire, la première dent secondaire et la deuxième dent secondaire s'étendent dans un même premier plan d'extension.

Selon une possibilité, le premier bras de liaison relie les bords externes de la première dent primaire et de la première dent secondaire et le deuxième bras de liaison relie les bords externes de la deuxième dent primaire et de la deuxième dent secondaire.

Selon une autre possibilité, la première fente et la deuxième fente s'étendent sensiblement dans un second plan d'extension, le second plan d'extension s'étendant entre le premier bras de liaison et le deuxième bras de liaison.

Selon une possibilité, le couteau de chasse ou de survie comprend un pont de matière s'étendant entre les première et deuxième dents primaires et les première et deuxième dents secondaires.

Avantageusement, le dispositif d'extraction de tique est mobile entre la position de rangement et la position d'utilisation suivant un mouvement de translation dans la direction d'extension du manche du couteau. Le dispositif est ainsi parfaitement protégé à l'intérieur du manche et les organes de retrait ne risquent pas de s'accrocher avec des éléments extérieurs au couteau, tel qu'avec les vêtements de l'utilisateur par exemple.

Selon une autre disposition, le couteau de chasse ou de survie comprend:
- un organe de dépeçage mobile par rapport au manche entre une position escamotée dans laquelle l'organe de dépeçage s'étend à l'intérieur du manche et une position déployée dans laquelle l'organe de dépeçage s'étend au moins en partie hors du manche,
- des moyens de liaison reliant l'organe de dépeçage et le dispositif d'extraction de tique, lesdits moyens de liaison étant configurés pour subordonner le déploiement de l'organe de dépeçage à la position du dispositif d'extraction et le déploiement du dispositif d'extraction à la position de l'organe de dépeçage.

Avantageusement, les moyens de liaison comprennent un premier élément de liaison rattaché à l'organe de dépeçage et un deuxième élément de liaison rattaché au dispositif d'extraction de tique, les premier et deuxième éléments de liaison étant configurés pour bloquer un déplacement du dispositif d'extraction de tique vers la position d'utilisation quand l'organe de dépeçage est en position escamotée ou en position déployée, et pour bloquer un déplacement de l'organe de dépeçage vers la position déployée ou vers la position escamotée quand le dispositif d'extraction de tique est en position de rangement.

Selon une caractéristique, les moyens de liaison comprennent une platine délimitant une rainure en U, et un ergot mobile à l'intérieur de la rainure en U.

Selon d'autres caractéristiques, le couteau de chasse ou de survie de l'invention comporte les caractéristiques optionnelles suivantes considérées seule ou en combinaison :
- Le premier bras de liaison et le deuxième bras de liaison se rejoignent au niveau de la portion de préhension.
- La portion de préhension présente une forme allongée.
- La portion de préhension s'étend dans le prolongement de la portion d'extrémité.
- Le premier plan d'extension s'étend de manière sensiblement perpendiculaire à l'axe d'extension de la portion de préhension.
- Le deuxième élément de liaison est rattaché à une deuxième portion d'extrémité du dispositif d'extraction de tique, opposée à la portion d'extrémité.
- La première bissectrice et la deuxième bissectrice s'étendent dans un même plan, s'étendant entre les deux bras de liaison.
- Le pont de matière s'étend au moins en partie dans le premier plan d'extension.
- Les bords internes des première et deuxième dents primaires et des première et deuxième dents secondaires sont biseautés ou chanfreinés
- Les extrémités avant des première et deuxième dents primaires et des première et deuxième dents secondaires sont courbes.
- Le dispositif d'extraction de tique est constitué en matière plastique rigide. Ceci le rend facile à nettoyer.

D'autres aspects, buts et avantages de la présente invention apparaîtront mieux à la lecture de la description suivante d'un mode de réalisation de celle-ci, donné à titre d'exemple non limitatif et fait en référence aux dessins annexés. Les figures ne respectent pas nécessairement l'échelle de tous les éléments représentés de sorte à améliorer leur lisibilité. Dans la suite de la description, par souci de simplification, des éléments identiques, similaires ou équivalents des différentes formes de réalisation portent les mêmes références numériques.
[Fig. 1] représente une vue schématique du couteau de chasse ou de survie illustrant le dispositif d'extraction de tique logé dans le manche, en position de rangement, selon un mode de réalisation de l'invention.
[Fig. 2] représente une vue schématique du couteau de chasse ou de survie illustrant le dispositif d'extraction de tique en position d'utilisation dans laquelle il s'étend hors du manche, selon un mode de réalisation de l'invention.
[Fig. 3] représente une vue schématique en perspective du dispositif d'extraction de tique en position d'utilisation, selon un mode de réalisation de l'invention.
[Fig. 4] représente une vue schématique d'un agrandissement des première et deuxième fentes du dispositif d'extraction de tique, selon un mode de réalisation de l'invention.
[Fig. 5] représente une vue schématique d'un agrandissement du dessous des première et deuxième fentes du dispositif d'extraction de tique, selon un mode de réalisation de l'invention.
[Fig. 6] représente une vue schématique d'une vue en coupe transversale du dispositif d'extraction de tique coopérant avec un organe de dépeçage du couteau de chasse ou de survie par l'intermédiaire d'un premier et deuxième moyens de liaison, selon un mode de réalisation de l'invention.
[Fig. 7] représente une vue schématique d'une vue en coupe transversale d'un premier et deuxième moyens de liaison en position intermédiaire selon un mode de réalisation de l'invention.
[Fig. 8] représente une vue schématique d'une vue en coupe transversale d'un premier et deuxième moyens de liaison en position centrale selon un mode de réalisation de l'invention.

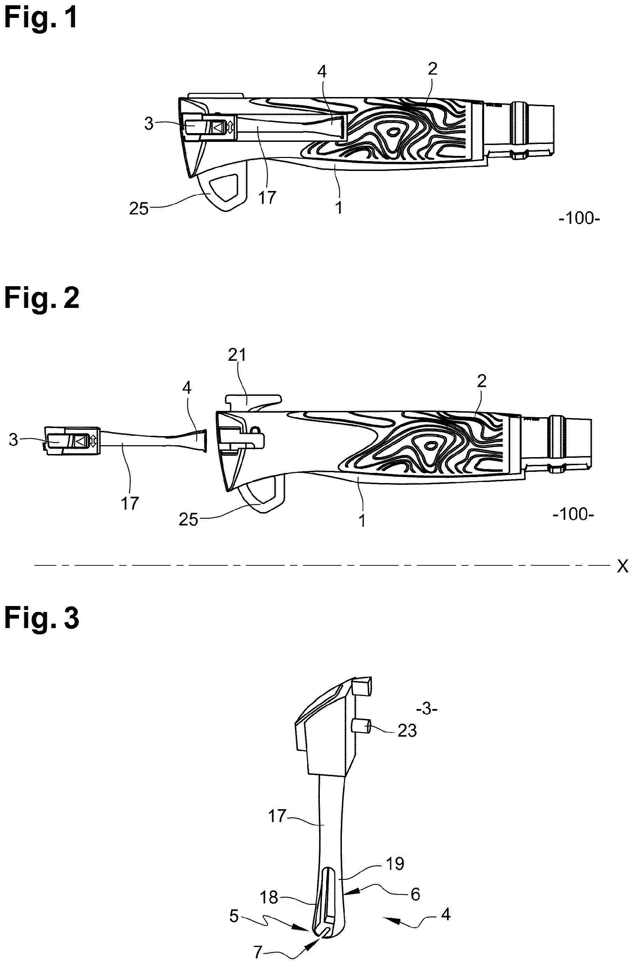

Comme illustré sur la figure 1, le couteau de chasse ou de survie 100 selon l'invention est un couteau multifonctionnel présentant à une région d'extrémité une lame 1 pouvant pivoter à l'intérieur du manche 2. A une région d'extrémité opposée est disposé le dispositif d'extraction de tique 3 logé de façon amovible à l'intérieur du manche 2 dans une position de rangement.

En référence à la figure 2 , le dispositif d'extraction de tique 3 est en position d'utilisation, dans laquelle il s'étend en dehors du manche 2. Le déplacement entre ses deux positions est réalisé selon un mouvement de translation dans la direction d'extension du manche 2 du couteau 100.

Les figures 2 et 3 illustrent une portion d'extrémité 4 du dispositif d'extraction de tique 3 sur laquelle sont juxtaposés un premier organe de retrait de tique 5 et un deuxième organe de retrait de tique 6, configurés pour le retrait de tique de tailles différentes. Les premier et deuxième organes de retrait de tique 5,6 présentent respectivement une première fente 7 configurée pour enserrer une tique d'une première taille et une deuxième fente 8 configurée pour enserrer une tique d'une deuxième taille de sorte que le dispositif 3 rend possible le retrait efficace de tique de tailles différentes sans les comprimer.

Egalement illustrées sur les figures 2 à 4, les première et deuxième fentes 7,8 comprennnent respectivement une première zone d'insertion 9 et une deuxième zone d'insertion 11, opposées l'une à l'autre, chacune des zones d'insertion 9,11 étant configurée pour l'insertion d'une taille particulière de tique de sorte à permettre une insertion de tique de différentes tailles dans un même dispositif d'extraction de tique 3.

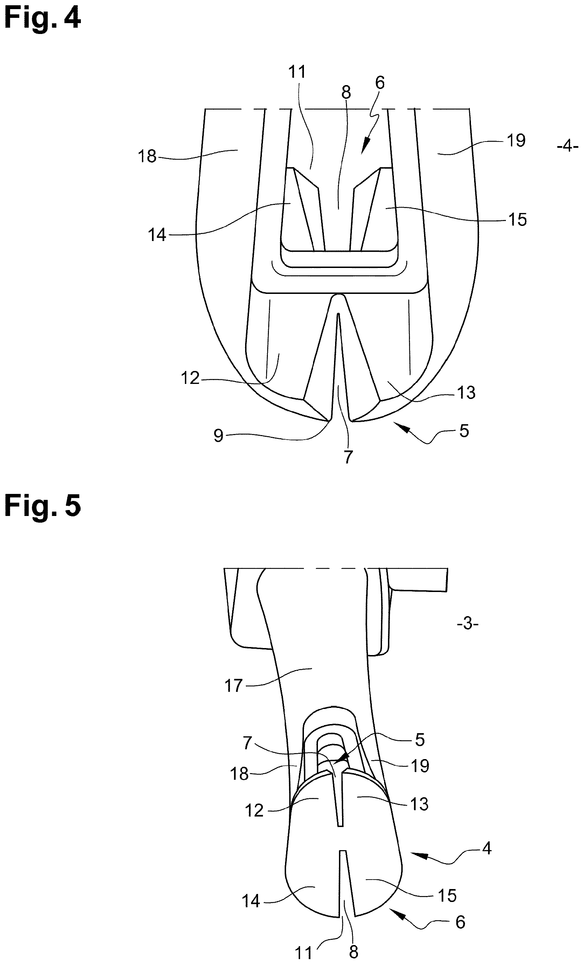

En référence aux figures 4 et 5, la première fente 7 présente une première forme en V délimitée par une première dent primaire 12 et une deuxième dent primaire 13 s'étendant dans un même premier plan d'extension. Réciproquement, la deuxième fente 8 présente une forme de V délimitée par une première dent secondaire 14 et une deuxième dent secondaire 15 s'étendant dans le même premier plan d'extension. La première forme en V et la deuxième forme en V s'étendent selon deux directions opposées. Un pont de matière 16 s'étend entre les premières et deuxième dents primaires 12,13 et les première et deuxième dents secondaires 14,15.

Selon une autre disposition illustrée à la figure 3, le dispositif d'extraction de tique 3 comprend une portion de préhension 17 s'étendant suivant l'axe d'extension X du dispositif d'extraction 3, dans le prolongement de la portion d'extrémité 4. Un premier bras de liaison 18 et un deuxième bras de liaison 19 relient le premier organe de retrait de tique 5 et le deuxième organe de retrait de tique 6 à la portion de préhension 17.

Comme illustré en détail aux figures 3 et 4, le premier bras de liaison 18 relie les bords externes de la première dent primaire 12 et de la première dent secondaire 14. Réciproquement, le deuxième bras de liaison 19 relie les bords externes de la deuxième dent primaire 13 et de la deuxième dent secondaire 15, de sorte à assurer la tenue mécanique de l'ensemble.

Comme on peut le voir figure 5, la première fente 7 et la deuxième fente 8 s'étendent dans un second plan d'extension qui s'étend entre le premier bras de liaison 18 et le deuxième bras de liaison 19.

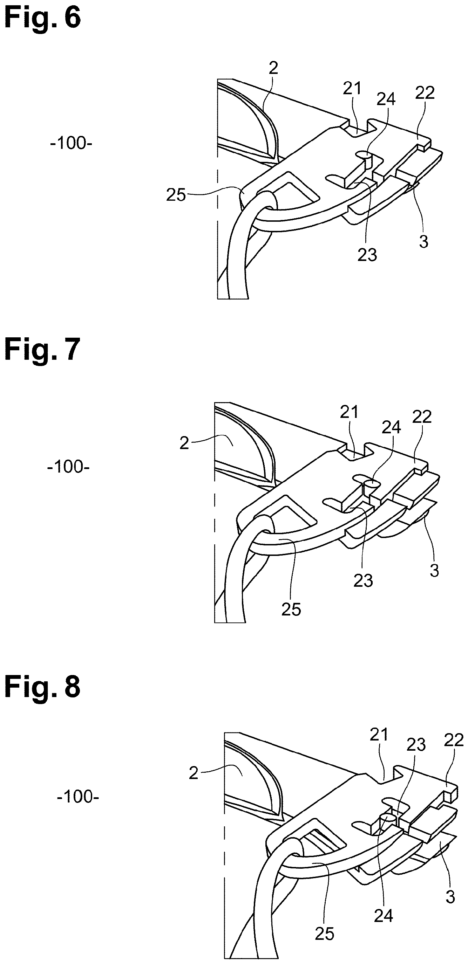

Selon une possibilité de réalisation du couteau de chasse ou de survie 100 de la présente invention illustrée à la figure 6, le couteau 100 selon l'invention comprend un organe de dépeçage 21 mobile par rapport au manche 2 entre une position déployée dans laquelle l'organe de dépecage 21 s'étend au moins en partie hors du manche 2 et une position escamotée dans laquelle l'organe de dépecage 21 s'étend à l'intérieur du manche 2 (figure 6). L'organe de dépeçage 21 est relié à un premier moyen de liaison constitué par une platine 22 comprenant une rainure 23 en U, qui coopère avec un deuxième moyen de liaison, constitué par un ergot 24 rattaché au dispositif d'extraction de tique 3 (l'ergot 24 est mobile à l'intérieur de la rainure 23 en U) de sorte que le déploiement de l'organe de dépeçage 21 est subordonné à la position du dispositif d'extraction de tique 3. Réciproquement, le déploiement du dispositif d'extraction de tique 3 est subordonné à la position de l'organe de dépeçage 21.

En effet, la platine 22 et l'ergot 24 coopèrent pour bloquer le déplacement du dispositif d'extraction de tique 3 vers la position d'utilisation quand l'organe de dépeçage 21 est en position déployée ou rétractée (figure 6). Et lorsque le dispositif d'extraction de tique 3 est en position de rangement, les moyens de liaison 23,24 bloquent le déplacement de l'organe de dépeçage 21 en position escamotée (figure 6) ou en position déployée (non illustrée). Dans une position intermédiaire de l'ergot 24 dans la rainure 23 (figure 7) l'organe de dépeçage 21 peut être déplacé entre la position déployée et la position rétractée. De même en position centrale de l'ergot 24 dans la rainure 23 (La platine est disposée de sorte que l'ergot 24 est disposé au centre des deux branches du U, figure 8) le dispositif d'extraction de tique 3 peut être déplacé dans une position d'utilisation.

Selon une disposition illustrée aux figures 1, 6 à 8, un anneau d'accroche 25 est prévu sur la platine 22, du coté opposé à l'organe de dépeçage 21. Le déplacement de cet anneau d'accroche 25 est subordonné à la position du dispositif d'extraction de tique 3, par un mécanisme similaire à celui qui régit le déplacement de l'organe de dépeçage 21, avec un mouvement en direction inverse.

Ainsi, la présente invention propose un couteau de chasse ou de survie 100 qui permet de loger efficacement un dispositif d'extraction de tique 3 double taille de sorte à pouvoir retirer des tiques de tailles différentes de façon sécuritaire, en position d'utilisation du dispositif d'extraction de tique 3.

Il va de soi que l'invention n'est pas limitée aux variantes de réalisation décrites ci-dessus à titre d'exemple mais qu'elle comprend tous les équivalents techniques et les variantes des moyens décrits ainsi que leurs combinaisons, tout en restant dans l'étendue de protection définie par les revendications suivantes.

## Revendications

1. Couteau de chasse ou de survie (100) comprenant un manche (2) et un dispositif d'extraction de tique (3) destiné à extraire une tique de la peau des animaux ou de l'homme, le dispositif d'extraction de tique (3) étant logé de façon amovible dans le manche (2), en étant mobile entre une position de rangement dans laquelle le dispositif d'extraction de tique (3) est logé à l'intérieur du manche (2) et une position d'utilisation dans laquelle le dispositif d'extraction de tique (3) s'étend hors du manche (2), le dispositif d'extraction de tique (3) comprenant une portion d'extrémité (4) sur laquelle sont juxtaposés :
- un premier organe de retrait de tique (5) comprenant une première fente (7) configurée pour enserrer une tique d'une première taille en vue de son retrait,
- un deuxième organe de retrait de tique (6) comprenant une deuxième fente (8) configurée pour enserrer une tique d'une deuxième taille en vue de son retrait, de telle sorte que les premier et deuxième organes de retrait de tique sont aptes au retrait de tiques de tailles différentes.

2. Couteau de chasse ou de survie (100) selon la revendication 1, dans lequel la première fente (7) et la deuxième fente (8) comprennent respectivement une première zone d'insertion (9) configurée pour l'insertion d'une tique d'une première taille et une deuxième zone d'insertion (11) configurée pour l'insertion d'une tique d'une deuxième taille, différente de la première taille, la deuxième zone d'insertion (11) étant opposée à la première zone d'insertion (9).

3. Couteau de chasse ou de survie (100) selon l'une des revendications 1 ou 2, dans lequel la première fente (7) et de la deuxième fente (8) présentent respectivement une première forme en V et une deuxième forme en V, s'étendant dans deux directions opposées.

4. Couteau de chasse ou de survie (100) selon l'une des revendications précédentes, dans lequel le dispositif d'extraction de tique (3) comprend une portion de préhension (17), un premier bras de liaison (18) et un deuxième bras de liaison (19) reliant le premier organe de retrait de tique (5) et le deuxième organe de retrait de tique (6) à la portion de préhension (17).

5. Couteau de chasse ou de survie (100) selon l'une des revendications précédentes, dans lequel le premier organe de retrait de tique (5) comprend une première dent primaire (12) et une deuxième dent primaire (13) délimitant la première fente (7) et dans lequel le deuxième organe de retrait de tique (6) comprend une première dent secondaire (14) et une deuxième dent secondaire (15) délimitant la deuxième fente (8).

6. Couteau de chasse ou de survie (100) selon les revendications 4 et 5, dans lequel le premier bras de liaison (18) relie les bords externes de la première dent primaire (12) et de la première dent secondaire (14) et le deuxième bras de liaison (19) relie les bords externes de la deuxième dent primaire (13) et de la deuxième dent secondaire (15).

7. Couteau de chasse ou de survie (100) selon l'une des revendications 5 à 6, dans lequel la première dent primaire (12), la deuxième dent primaire (13), la première dent secondaire (14) et la deuxième dent secondaire (15) s'étendent dans un même premier plan d'extension.

8. Couteau de chasse ou de survie (100) selon l'une des revendications 4 à 7, dans lequel la première fente (7) et la deuxième fente (8) s'étendent sensiblement dans un second plan d'extension, le second plan d'extension s'étendant entre le premier bras de liaison et le deuxième bras de liaison (19).

9. Couteau de chasse ou de survie (100) selon l'une des revendications 5 à 8, lequel comprend un pont de matière (16) s'étendant entre les première et deuxième dents primaires (12,13) et les première et deuxième dents secondaires (14,15).

10. Couteau de chasse ou de survie (100) selon l'une des revendications précédentes, dans lequel le dispositif d'extraction de tique (3) est mobile entre la position de rangement et la position d'utilisation suivant un mouvement de translation dans la direction d'extension du manche (2) du couteau.

## Patentansprüche

1. Jagd- oder Survivalmesser (100), das einen Griff (2) und eine Zeckenentfernungsvorrichtung (3) umfasst, die dazu bestimmt ist, eine Zecke aus der Haut von Tieren oder eines Menschen zu entfernen, wobei die Zeckenentfernungsvorrichtung (3) herausnehmbar im Griff (2) untergebracht ist, indem sie zwischen einer Aufbewahrungsposition, in der die Zeckenentfernungsvorrichtung (3) im Inneren des Griffs (2) untergebracht ist, und einer Gebrauchsposition, in der sich die Zeckenentfernungsvorrichtung (3) außerhalb des Griffs (2) erstreckt, beweglich ist, wobei die Zeckenentfernungsvorrichtung (3) einen Endabschnitt (4) umfasst, an dem sich nebeneinander befinden:
- ein erstes Zeckenziehorgan (5), das einen ersten Schlitz (7) umfasst, der dafür konfiguriert ist, eine Zecke einer ersten Größe zu deren Ziehen zu umschließen,
- ein zweites Zeckenziehorgan (6), das einen zweiten Schlitz (8) umfasst, der dafür konfiguriert ist, eine Zecke einer zweiten Größe zu deren Ziehen zu umschließen, sodass das erste und das zweite Zeckenziehorgan zum Ziehen von Zecken unterschiedlicher Größen geeignet sind.

2. Jagd- oder Survivalmesser (100) nach Anspruch 1, wobei der erste Schlitz (7) und der zweite Schlitz (8) jeweils einen ersten Einführbereich (9), der zum Einführen einer Zecke einer ersten Größe konfiguriert ist, und einen zweiten Einführbereich (11), der zum Einführen einer Zecke einer zweiten Größe konfiguriert ist, die sich von der ersten Größe unterscheidet, umfassen, wobei der zweite Einführbereich (11) dem ersten Einführbereich (9) gegenüberliegt.

3. Jagd- oder Survivalmesser (100) nach einem der Ansprüche 1 oder 2, wobei der erste Schlitz (7) und der zweite Schlitz (8) jeweils eine erste V-Form und eine zweite V-Form aufweisen, die sich in zwei entgegengesetzte Richtungen erstrecken.

4. Jagd- oder Survivalmesser (100) nach einem der vorstehenden Ansprüche, wobei die Zeckenentfernungsvorrichtung (3) einen Greifabschnitt (17) umfasst, wobei ein erster Verbindungsarm (18) und ein zweiter Verbindungsarm (19) das erste Zeckenziehorgan (5) und das zweite Zeckenziehorgan (6) mit dem Greifabschnitt (17) verbinden.

5. Jagd- oder Survivalmesser (100) nach einem der vorstehenden Ansprüche, wobei das erste Zeckenziehorgan (5) einen ersten Primärzahn (12) und einen zweiten Primärzahn (13) umfasst, die den ersten Schlitz (7) begrenzen, und wobei das zweite Zeckenziehorgan (6) einen ersten Sekundärzahn (14) und einen zweiten Sekundärzahn (15) umfasst, die den zweiten Schlitz (8) begrenzen.

6. Jagd- oder Survivalmesser (100) nach den Ansprüchen 4 und 5, wobei der erste Verbindungsarm (18) die Außenkanten des ersten Primärzahns (12) und des ersten Sekundärzahns (14) verbindet, und der zweite Verbindungsarm (19) die Außenkanten des zweiten Primärzahns (13) und des zweiten Sekundärzahns (15) verbindet.

7. Jagd- oder Survivalmesser (100) nach einem der Ansprüche 5 bis 6, wobei sich der erste Primärzahn (12), der zweite Primärzahn (13), der erste Sekundärzahn (14) und der zweite Sekundärzahn (15) in derselben ersten Erstreckungsebene erstrecken.

8. Jagd- oder Survivalmesser (100) nach einem der Ansprüche 4 bis 7, wobei sich der erste Schlitz (7) und der zweite Schlitz (8) im Wesentlichen in einer zweiten Erstreckungsebene erstrecken, wobei sich die zweite Erstreckungsebene zwischen dem ersten Verbindungsarm und dem zweiten Verbindungsarm (19) erstreckt.

9. Jagd- oder Survivalmesser (100) nach einem der Ansprüche 5 bis 8, das eine Materialbrücke (16) umfasst, die sich zwischen dem ersten und dem zweiten Primärzahn (12, 13) und dem ersten und dem zweiten Sekundärzahn (14, 15) erstreckt.

10. Jagd- oder Survivalmesser (100) nach einem der vorstehenden Ansprüche, wobei die Zeckenentfernungsvorrichtung (3) zwischen der Aufbewahrungsposition und der Gebrauchsposition gemäß einer Verschiebebewegung in der Erstreckungsrichtung des Griffs (2) des Messers beweglich ist.

## Claims

1. A hunting or survival knife (100) comprising a handle (2) and a tick extraction device (3) intended to extract a tick from the skin of animals or humans, the tick extraction device (3) being removably housed in the handle (2), by being movable between a storage position in which the tick extraction device (3) is housed inside the handle (2) and a use position in which the tick extraction device (3) extends out of the handle (2), the tick extraction device (3) comprising an end portion (4) on which are juxtaposed:
- a first member for removing the tick (5) comprising a first slot (7) configured to grip a tick of a first size for its removal,
- a second member for removing the tick (6) comprising a second slot (8) configured to grip a tick of a second size for its removal, such that the first and second tick removal members are capable of removing ticks of different sizes.

2. The hunting or survival knife (100) according to claim 1, wherein the first slot (7) and the second slot (8) respectively comprise a first insertion zone (9) configured for the insertion of a tick of a first size and a second insertion zone (11) configured for the insertion of a tick of a second size, different from the first size, the second insertion zone (11) being opposite the first insertion zone (9).

3. The hunting or survival knife (100) according to any of claims 1 or 2, wherein the first slot (7) and the second slot (8) respectively have a first V shape and a second V shape, extending in two opposite directions.

4. The hunting or survival knife (100) according to any of the preceding claims, wherein the tick extraction device (3) comprises a gripping portion (17), a first connecting arm (18) and a second connecting arm (19) connecting the first tick removal member (5) and the second tick removal member (6) to the gripping portion (17).

5. The hunting or survival knife (100) according to any of the preceding claims, wherein the first tick removal member (5) comprises a first primary tooth (12) and a second primary tooth (13) delimiting the first slot (7) and in which the second tick removal member (6) comprises a first secondary tooth (14) and a second secondary tooth (15) delimiting the second slot (8).

6. The hunting or survival knife (100) according to claims 4 and 5, wherein the first connecting arm (18) connects the external edges of the first primary tooth (12) and the first secondary tooth (14) and the second connecting arm (19) connects the external edges of the second primary tooth (13) and the second secondary tooth (15).

7. The hunting or survival knife (100) according to any of claims 5 to 6, wherein the first primary tooth (12), the second primary tooth (13), the first secondary tooth (14) and the second secondary tooth (15) extend in the same first extension plane.

8. The hunting or survival knife (100) according to any of claims 4 to 7, wherein the first slot (7) and the second slot (8) extend substantially in a second extension plane, the second extension plane extending between the first connecting arm and the second connecting arm (19).

9. The hunting or survival knife (100) according to any of claims 5 to 8, which comprises a bridge of material (16) extending between the first and second primary teeth (12, 13) and the first and second secondary teeth (14, 15).

10. The hunting or survival knife (100) according to any of the preceding claims, wherein the tick extraction device (3) is movable between the storage position and the use position following a translation movement in the direction of extension of the handle (2) of the knife.
